(19) Europäisches Patentamt
European Patent Office
Office européen des brevets

(11) EP 1 158 954 B1

(12) **EUROPÄISCHE PATENTSCHRIFT**

(45) Veröffentlichungstag und Bekanntmachung des Hinweises auf die Patenterteilung:
**16.06.2004 Patentblatt 2004/25**

(21) Anmeldenummer: **00985176.7**

(22) Anmeldetag: **16.12.2000**

(51) Int Cl.$^7$: **A61K 7/13**

(86) Internationale Anmeldenummer:
**PCT/EP2000/012847**

(87) Internationale Veröffentlichungsnummer:
**WO 2001/051019 (19.07.2001 Gazette 2001/29)**

# (54) MITTEL UND VERFAHREN ZUR FÄRBUNG KERATINISCHER FASERN

MEANS AND METHOD FOR DYING KERATINIC FIBRES

AGENT POUR COLORER DES FIBRES KERATINIQUES

(84) Benannte Vertragsstaaten:
**AT BE CH CY DE DK ES FI FR GB GR IE IT LI LU MC NL PT SE TR**

(30) Priorität: **07.01.2000 DE 10000460**

(43) Veröffentlichungstag der Anmeldung:
**05.12.2001 Patentblatt 2001/49**

(73) Patentinhaber: **Wella Aktiengesellschaft
64274 Darmstadt (DE)**

(72) Erfinder: **BRAUN, Hans-Jürgen
CH-3182 Überstorf (CH)**

(56) Entgegenhaltungen:
**WO-A-99/29285**
**WO-A-99/66890**
**DE-A- 19 757 510**
**US-A- 5 980 584**

- **DATABASE WPI Section Ch, Week 198031 Derwent Publications Ltd., London, GB; Class D21, AN 1980-54659C XP002163850 BRISTOL-MYERS CO.: "3-(2,4-Diaminophenoxy)-1,2-propane diol - useful as oxidation coupler for hair dyes" & RESEARCH DISCLOSURE, Bd. 195, Nr. 013, 10. Juli 1980 (1980-07-10), EMSWORTH, GB**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft eine Zusammensetzung zur Färbung von keratinischen Fasern, insbesondere von menschlichen Haaren, mit einem Gehalt an 3-(2,4-Diaminophenoxy)-1-propanol sowie mindestens einem p-Phenylendiaminderivat, 4,5-Diamino-1H-pyrazolderivat oder p-Aminophenolderivat sowie ein Verfahren zur Färbung keratinischer Fasern.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines Oxidationsmittels.

An Oxidationsfarbstoffe, die zur Färbung menschlicher Haare verwendet werden sollen, sind zahlreiche besondere Anforderungen gestellt. Die Farbstoffe müssen in toxikologischer und dermatologischer Hinsicht unbedenklich sowie nicht sensibilisierend sein und Färbungen in der gewünschten Intensität ermöglichen.

Die Haarfärbungen sollen für mindestens vier bis sechs Wochen stabil bleiben. Es wird auch erwartet, daß die Haarfärbungen bei der Anwendung von weiteren kosmetischen Behandlungen wie Shampoonieren oder Haarumformung stabil bleiben. Im weiteren sollen die Haarfärbungen auch stabil sein gegen äußere Einwirkungen wie Licht und Wetter sowie gegenüber Schweiß oder mechanischem Abrieb.

Außerdem ist es erforderlich, daß durch Kombination geeigneter Entwicklersubstanzen und Kupplersubstanzen eine breite Palette verschiedener Farbnuancen erzeugt werden kann.

Ein besonderes Problem stellt die Erzeugung von stabilen blauen und rötlichen Farbtönen dar. Blaufärbende HaarEarbkuppler werden zusammen mit gelben und roten Farbkomponenten zur Erzeugung von natürlich wirkenden Haarfärbungen benötigt. Modische rötliche Haarfärbungen werden dagegen durch Verwendung eines Überschusses an roten Farbkomponenten erzeugt.

Die Stabilität der Haarfärbungen sowohl im Blaubereich als auch im Rotbereich läßt bei herkömmlichen Haarfärbemitteln besonders hinsichtlich der Einwirkung von organischen Säuren beziehungsweise Schweiß jedoch noch viele Wünsche offen.

Es wurde nun überraschend gefunden, daß mit einer Kombination bestehend aus der Kupplersubstanz 3-(2,4-Diaminophenoxy)-1-propanol und mindestens einer Entwicklersubstanz aus der Gruppe bestehend aus bestimmten p-Phenylendiaminderivaten, 4,5-Diamino-1H-pyrazolderivaten und p-Aminophenolderivaten Haarfärbemittel erhalten werden, die neben einer hervorragenden Waschstabilität und Lichtstabilität eine gegenüber dem Stand der Technik wesentlich verbesserte Stabilität gegenüber der Einwirkung von organischen Säuren beziehungsweise Schweiß aufweisen.

Die Darstellung von 3-(2,4-Diaminophenoxy)-1-propanol-dihydrochlorid sowie dessen Verwendung in Haarfärbemitteln ist aus der US-PS 4 259 261 und US-PS 4 329 504 bekannt.

Gegenstand der vorliegenden Erfindung ist ein Mittel zur Färbung von Keratinfasern, insbesondere menschlichen Haaren, auf der Basis einer Entwicklersubstanz-Kupplersubstanz-Kombination, welches dadurch gekennzeichnet ist, dass es als Kupplersubstanz 3-(2,4-Diaminophenoxy)-1-propanol und als Entwicklersubstanz

- ein am Benzolring monosubstituiertes p-Phenylendiaminderivat der allqemeinen Formel (1),

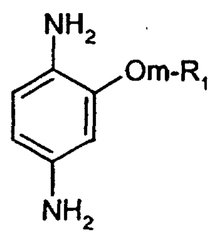

(I)

,

worin m gleich 0 oder 1 ist und **$R_1$** eine geradkettigen oder verzweigten $C_3$-$C_6$-Alkylrest, einen geradkettigen oder verzweigten Monohydroxy-($C_1$-$C_6$)-alkylrest oder Polyhydroxy-($C_2$-$C_6$)-alkylrest, einen geradkettigen oder verzweigten Mono-($C_2$-$C_6$)-alkoxy-($C_2$-$C_6$)-alkylrest oder Poly-($C_2$-$C_6$)-alkoxy-($C_2$-$C_6$)-alkylrest oder eine carbozyklischen oder heterozyklische, substituierte oder unsubstituierte, aromatische Verbindung darstellt; und/oder

- ein 4,5-Diamino-1H-Pyrazolderivat gemäß der allgemeinen Formel (II)

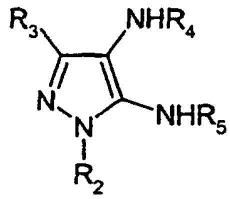

(II)

,

worin $R_2$, $R_4$ und $R_5$ unabhängig voneinander ein Wasserstoffatom oder einen geradkettigen oder verzweigten $C_1$-$C_6$-Alkylrest, einen geradkettigen oder verzweigten Monohydroxy-($C_1$-$C_6$)-alkylrest oder Polyhydroxy-($C_2$-$C_6$)-alkylrest oder einen unsubstituierten oder am Aromaten substituierten Benzylrest darstellen, und $R_3$ gleich einem geradkettigen oder verzweigten $C_1$-$C_6$-Alkylrest, einem geradkettigen oder verzweigten Monohydroxy-($C_1$-$C_6$)-alkylrest oder Polyhydroxy-($C_2$-$C_6$)-alkylrest, einem geradkettigen oder verzweigten Mono-($C_2$-$C_6$)-alkoxy-($C_2$-$C_6$)-alkylrest oder Poly-($C_2$-$C_6$)-alkoxy-($C_2$-$C_6$)-alkylrest oder einer carbozyklischen oder heterozyklischen, substituierten oder unsubstituierten, aromatischen Verbindung ist, oder $R_2$ gleich einer Methylgruppe, einer Isopropylgruppe, einer 2-Hydroxyethylgruppe, einer Benzylgruppe oder einer 4-Methyl-benzylgruppe ist, wenn gilt $R_3$=$R_4$=$R_5$ Wasserstoff;
und/oder

- ein p-Aminophenolderivat der allgemeinen Formel (III)

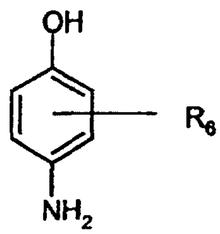

(III)

.

worin $R_6$ gleich einem geradkettigen oder verzweigten $C_1$-$C_6$-Alkylrest, einem geradkettigen oder verzweigten Monohydroxy-($C_1$-$C_6$)-alkylrest oder Polyhydroxy-($C_2$-$C_6$)-alkylrest, einem geradkettigen oder verzweigten Mono-($C_2$-$C_6$)-alkoxy-($C_2$-$C_6$)-alkylrest oder Poly-($C_2$-$C_6$)-alkoxy-($C_2$-$C_6$)-alkylrest, einem geradkettigen oder verzweigten Amino-($C_2$-$C_6$)-Alkylrest oder einer carbozyklischen oder heterozyklischen, substituierten oder unsubstituierten, aromatischen Verbindung ist, enthält.

Bevorzugte p-Phenylendiaminderivate der Formel (I) sind 2-Propyl-p-phenylendiamin, 2-(Hydroxymethyl)-p-phenylendiamin, 2-(1-Hydroxyethyl)-p-phenylendiamin, 2-(2-Hydroxyethyl)-p-phenylendiamin, 2-(3-Hydroxypropyl)-p-phenylendiamin, 2-(1,2-Dihydroxyethyl)-pphenylendiamin, 2-(Methoxymethyl)-p-phenylendiamin, 2-(2-Methoxyethyl)-p-phenylendiamin, 2-(2-Hydroxyethoxy)-p-phenylendiamin, 2,5-Diaminobiphenyl, 2-(2-Thienyl)-p-phenylendiamin, 2-(3-Thienyl)-pphenylendiamin und 3-(2,5-Diaminophenyl)pyridin.

Bevorzugte Pyrazolderivate der Formel (11) sind 4,5-Diamino-1-methyl-1H-pyrazol, 4,5-Diamino.-1-(2-hydroxy-ethyl)-1H-pyrazol, 4,5-Diamino-1-(-1-methylethyl)-1H-pyrazol, 4,5-Diamino-1-(phenylmethyl)-1H-pyrazol, 4,5-Diamino-1-((4-methylphenyl)methyl)-1H-pyrazol, 4,5-Diamino-1-(2-hydroxyethyl)-3-methyl-1H-pyrazol und 4,5-Diamino-1-methyl-3-phenyl-1H-pyrazol.

Bevorzugte p-Aminophenolderivate der Formel (III) sind 3-Methyl-p-aminophenol, 2-Aminomethyl-p-aminophenol, 2-Hydroxymethyl-p-amino-phenol, 3-Hydroxymethyl-p-amino-phenol, 2-Phenyl-p-aminophenol, 2-(2-Thienyl)-p-aminophenol, 2-(3-Thienyl)-p-aminophenol, 3-(2-Thienyl)-p-aminophenol und 3-(3-Thienyl)-p-aminophenol.

Zur Abrundung des Farbergebnisses sowie zur Erzeugung von speziellen Farbeffekten können den erfindungsgemäßen Kombinationen weitere Entwicklersubstanzen und/oder Kupplersubstanzen sowie direktziehende Farbstoffe zugesetzt werden.

Als Entwicklersubstanzen können beispielsweise die folgenden Verbindungen genannt werden: 1,4-Diaminobenzol; 1,4-Diamino-2-methylbenzol; 1,4-Diamino-2,6-dimethytbenzol; 2,5-Diamino-1,3-diethylbenzol, 1,4-Diamino-2,5-dimethylbenzol; 1,4-Diamino-2,3-dimethyl-benzol; 2-Chlor-1,4-diamino-benzol; 1-(Aminomethyl)-2,5-diaminobenzol, 2-(2-(Acetylamino)ethoxy)-1,4-diaminobenzol, 4-Phenylamino-anilin; 4-Dimethylamino-anilin; 4-(Dipropylamino)anilin; 4-Diethylamino-anilin; 4-(Ethyl(2-hydroxyethyl)amino)anilin; 4-(Di(2-hydroxyethyl)amino)-anilin; 4-(Di(2-hydroxyethyl)amino)-2-methylanilin; 4-((2-Methoxyethyl)amino)-anilin; 4-((3-Hydroxypropyl)amina)-anilin; 4-((2,3-Dihydroxypropyl)-amino)anilin; 1,3-Bis-((4-aminophenyl)(2-hydroxyethyl)amino)-2-propanol; 1,4-Di((4-Aminophenyl)amino)butan; 1,8-Bis-(2,5-diaminophenoxy)-3,6-dioxaoctan; 4-Aminophenol; 4-Amino-3-fluorphenol; 4-Methylaminophenol; 4-Amino-2-(aminomethyl)-phenol; 4-Amino-2-(hydroxymethyl)-phenol; 4-Amino-2-fluorphenol; 4-Amino-2-((2-hydroxyethyl)amino)methylphenol; 4-Amino-2-methylphenol; 4-Amino-2-(methoxymethyl)-phenol; 4-Amino-2-(2-hydroxyethyl)-phenol; 5-Amino-salicylsäure; 2,5-Diamino-pyridin; 2,4,5,6-Tetraamino-pyrimidin und 2,5,6-Triamino-4(1H)-pyrimidon.

Als Kupplersubstanzen können beispielsweise die folgenden Verbindungen genannt werden: 2,6-Diaminopyridin, 2-Amino-4-[(2-hydroxyethyl)-amino]-anisol, 2,4-Diamino-1-fluor-5-methyl-benzol, 2,4-Diamino-1-methoxy-5-methyl-benzol, 2,4-Diamino-1-ethoxy-5-methyf-benzol, 2.4-Diamino-1-(2-hydroxyethoxy)-5-methyl-benzol, 2,4-Di[(2-hydroxyethyl)-amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylaminophenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlorphenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphtha(in, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor- 3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)-amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxyindol,7-Hydroxy-indol und 2,3-lndolindion.

Weiterhin kann das erfindungsgemäße Färbemittel zusätzlich andere Farbkomponenten, wie zum Beispiel 2-Aminophenol, 2-Amino-6-methylphenol oder 2-Amino-5-methylphenol, enthalten.

Als direktziehenden Farbstoffe können beispielsweise die folgenden Verbindungen genannt werden: 4-[(4'-Aminophenyl)-(4'imino-2",5"cyclohexadien-1"-yliden)-methyl]-2-methylaminobenzol-monohydrochlorid (C.I. 42 510), 4-[(4'-Amino-3'-methyl-phenyl)-(4"-imino-3"-methyl-2",5"cyclohexadien-1"-yliden)-methyl]-2-methyl-aminobenzol monohydrochlorid (C.I. 42 520), 4-(2'-Hydroxy-ethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl)-2-nitroanilin, 5-Chlor-2-hydroxy-4-nitroanilin, 2-Amino-4-chlor-6-nitrophenol, 1-[(2'-Ureidoethyl)amino-4-nitrobenzol, 6-[(4'-Aminophenyl)azo]-5hydroxynaphthalin-1-sulfonsäure-Natriumsalz (C.I. 14 805), 1,4-Diaminoanthrachinon und 1,4,5,8-Tetraaminoantrachinon.

Besonders bevorzugt sind Haarfärbemittel, welche eine der folgenden erfindungsgemässen Entwicklersubstanz-Kupplersubstanz-Kombinationen enthalten:

- 3-(2,4-Diaminophenoxy)-1-propanol und 2-(1-Hydroxyethyl)-p-phenylendiamin;
- 3-(2,4-Diaminophenoxy)-1-propanol und 2-(2-Hydroxyethyl)-p-phenylendiamin;
- 3-(2,4-Diaminophenoxy)-1-propanol und 2-Propyl-p-phenylendiamin;
- 3-(2,4-Diaminophenoxy)-1-propanol und 2-(3-Hydroxypropyl)-p-phenylendiamin;
- 3-(2,4-Diaminophenoxy)-1-propanol und 2-(1,2-Dihydroxyethyl)-p-phenylendiamin;
- 3-(2,4-Diaminophenoxy)-1-propanol und 2-(Methoxymethyl )-p-phenylendiamin;
- 3-(2 ,4-Diaminophenoxy)-1-propanol und 2-(2-Methoxyethyl)-p-phenylendiamin;
- 3-(2,4-Diaminophenoxy)-1-propanol und 2-(2-Hydroxyethoxy)-p-phenylendiamin;
- 3-(2,4-Diaminophenoxy)-1-propanol und 2,5-Diaminobiphenyl;
- 3-(2,4-Diaminophenoxy)-1-propanol und 2-(2-Thienyl)-p-phenylendiamin;
- 3-(2,4-Diaminophenoxy)-1-propanol und 2-(3-Thienyl)-p-phenylendiamin;
- 3-(2,4-Diaminophenoxy)-1-propanol und 3-(2,5-Diaminophenyl)-pyridin;
- 3-(2,4-Diaminophenoxy)-1-propanol und 4,5-Diamino-1-methyl-1H-pyrazol;
- 3-(2,4-Diaminophenoxy)-1-propanol und 4,5-Diamino-1-(2-hydroxyethyl)-1H-pyrazol;
- 3-(2,4-Diaminophenoxy)-1-propanol und 4,5-Diamino-1-(1-methylethyl)-1H-pyrazol;
- 3-(2,4-Diaminophenoxy)-1-propanol und 4,5-Diamino-1-(phenylmethyl)1H-pyrazol;
- 3-(2,4-Diaminophenoxy)-1-propanol und 4,5-Diamino-1-((4-methylphenyl)methyl)-1H-pyrazol;

- 3-(2,4-Diaminophenoxy)-1-propano) und 4,5-Diamino-1-(2-hydroxyethyl)-3-methyl-1H-pyrazol;
- 3-(2,4-Diaminophenoxy)-1-propanol und 4,5-Diamino-1-methyl-3-phenyl-1H-pyrazol;
- 3-(2,4-Diaminophenoxy)-1-propanol und 3-Methyl-p-aminophenol;
- 3-(2,4-Diaminophenoxy)-1-propanol und 2-Aminomethyl-p-aminophenol;
- 3-(2,4-Diaminophenoxy)-1-propanol und 2-Phenyl-p-aminophenol;
- 3-(2,4-Diaminophenoxy)-1-propanol und 2-(2-Thienyl)-p-aminophenol;
- 3-(2,4-Diaminophenoxy)-1-propanol und 2-(3-Thienyl)-p-aminophenol;
- 3-(2,4-Diaminophenoxy)-1-propanol und 3-(2-Thienyl)-p-aminophenol;
- 3-(2,4-Diaminophenoxy)-1-propanol und 3-(3-Thienyl)-p-aminophenol;
- 3-(2,4-Diaminophenoxy)-1-propanol und 2-(2-Hydroxyethyl)-p-phenylendiamin und 3-Methyl-p-aminophenol;
- 3-(2,4-Diaminophenoxy)-1-propanol und 2-(2-Hydroxyethyl)-p-phenylendiamin und 4,5-Diamino-1-(2-hydroxyethyl)-1H-pyrazol;
- 3-(2,4-Diaminophenoxy)-1-propanol und 2-Methyl-p-phenylendiamin und 3-Methyl-p-aminophenol;
- 3-(2,4-Diaminophenoxy)-1-propanol und 2-Methyl-p-phenylendiamin und 4,5-Diamino-1-(2-hydroxyethyl)-1H-pyrazol;
- 3-(2,4-Diaminophenoxy)-1-propanol und 2-Methoxymethyl-p-phenylendiamin und 3-Methyl-p-aminophenol;
- 3-(2,4-Diaminophenoxy)-1-propanol und 2-Methoxymethyl-p-phenylendiamin und 4,5-Diamino-1-(2-hydroxyethyt)-1H-pyrazol;
- 3-(2,4-Diaminophenoxy)-1-propanol und 2-(2-Thienyl)-p-phenylendiamin und 3-Methyl-p-aminophenol;
- 3-(2,4-Diaminophenoxy)-1-propanol und 2-(2-Thienyl)-p-phenylendiamin und 4,5-Diamino-1-(2-hydroxyethyl)-1H-pyrazol;
- 3-(2,4-Diaminophenoxy)-1-propanol und 2,5-Diamino-1,1'-biphenyl und 3-Methyl-p-aminophenol;
- 3-(2,4-Diaminophenoxy)-1-propanol und 2,5-Diamino-1,1'-biphenyl und 4,5-Diamino-1-(2-hydroxyethyl)-1H-pyrazol;
- 3-(2,4-Diaminophenoxy)-1-propanol und 2-(2-Hydroxyethoxy-p-phenylendiamin und 3-Methyl-p-aminophenol;
- 3-(2,4-Diaminophenoxy)-1-propanol und 2-(2-Hydroxyethoxy-p-phenylendiamin und 4,5-Diamino-1-(2-hydroxyethyl)-1H-pyrazol;
- 3-(2,4-Diaminophenoxy)-1-propanol und N,N-Bis(2-hydroxyethyl)-p-phenylendiamin und 4,5-Diamino-1-(2-hydroxyethyl)-1H-pyrazol;
- 3-(2,4-Diaminophenoxy)-1-propanol und p-Phenylendiamin und 4,5-Diamino-1-(2-hydroxyethyl)-1H-pyrazol.

Die vorgenannten erfindungsgemässen Kombinationen können noch einen oder mehrere zusätzliche Kuppler, insbesondere Resorcin, 2-Methylresorcin, 3-Aminophenol, 5-Amino-2-methylphenol, 3-Amino-2-chlor-6-methylphenol oder 1-Naphthol, enthalten.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Das erfindungsgemäße Haarfärbemittel enthält das 3-(2,4-Diaminophenoxy)-1-propanol sowie die Verbindungen der Formel (I) bis (III) jeweils in einer Menge von etwa 0,01 bis 5 Gewichtsprozent, vorzugsweise 0,01 bis 3,0 Gewichtsprozent, wobei die Gesamtmenge an Entwicklersubstanzen und Kupplersubstanzen in dem erfindungsgemässen Mittel etwa 0,1 bis 10 Gewichtsprozent beträgt.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des erfindungsgemässen Färbemittels kann beispielsweise eine Lösung, insbesondere eine wässrige oder wässrigalkoholische Lösung, eine Creme, ein Gel, ein Aerosolschaum oder eine Emulsion sein, wobei die verwendung in Form einer Creme, eines Geles oder einer Emulsion besonders bevorzugt ist. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30

Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Färbemittel schwach sauer, neutral oder alkalisch reagieren. Insbesondere weist es einen pH-Wert von 6,8 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einsteltung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3- bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugsweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Das erfindungsgemäße Haarfärbemittel zeichnet sich durch eine ausgezeichnete physiologische Verträglichkeit aus und ermöglicht Haarfärbungen mit ausgezeichneter Farbechtheit, insbesondere was die Lichtechtheit, Waschechtheit und Reibeechtheit sowie die Stabilität gegenüber der Einwirkung von Säuren beziehungsweise der Absonderung von Schweiß anbetrifft. Weiterhin hat sich gezeigt, dass solche Kombinationen, welche das Sulfatsalz des 3-(2,4-Di-aminophenoxy)-1-propanols enthalten, besonders stabile Haarfärbungen ergeben.Hinsichtlich der färberischen Eigenschaften bieten die erfindungsgemäßen Haarfärbemittel je nach Art und Zusammensetzung der Farbkomponenten eine breite Palette verschiedener Farbnuancen, welche sich von blonden über braune, purpume, violette bis hin zu blauen und schwarzen Farbtönen erstreckt. Hierbei zeichnen sich die Farbtöne durch ihre besondere Farbintensität aus. Die sehr guten färberischen Eigenschaften der Haarfärbemittel gemäß der vorliegenden Anmeldung zeigen sich weiterhin darin, daß diese Mittel eine Anfärbung von ergrauten, chemisch nicht vorgeschädigten Haaren problemlos und mit guter Deckkraft ermöglichen.

Die nachfolgenden Beispiele sollen den Gegenstand der Erfindung näher erläutern, ohne ihn darauf zu beschränken.

## Beispiele

### Synthesebeispiel

**Beispiel 1: Herstellung von 3-(2,4-Diaminophenoxy)-1-propanol-sulfat**

10 g 2,4-Dinitro-1-(3-hydroxypropoxy)-benzol werden in 100 ml Ethanol an 1 g Palladium 10% bei 5 bar Wasserstoffdruck hydriert. Nach 6 Stunden wird der Katalysator unter Inertgas entfernt und die erhaltene Lösung in 50 ml Tetrahydrofuran/3 ml konz. Schwefelsäure eingerührt. Der entstehende Niederschlag wird abfiltriert und 1 Stunde bei Raumtemperatur in Tetrahydrofuran supendiert. Absaugen und Trocknen liefert 6,5 g eines farblosen Produkts mit einem Schmelzpunkt von 190 bis 192 °C.

$^1$H-NMR (DMSO): 1,83 ppm (dt , $^3J_{HH}$ = 6,1 Hz, $^3_{HH}$ = 6,4 Hz, 2H); 3,57 ppm (t, $^3J_{HH}$ = 6,,1 Hz, 2H); 3,99 ppm (t, $^3J_{HH}$ = 6,4 Hz, 2H); 6,45 ppm (dd, $^3J_{HH}$ = 8,4 Hz, $^4J_{HH}$ = 2,6 Hz, 1H); 6,58 ppm (d, $^4J_{HH}$ = 2,6 Hz, 1H); 6,82 ppm (d, $^3J_{HH}$ = 8,4 Hz, 1H); 9,4 ppm (s, breit, 3H)

**Beispiele für Färbemittel**

**Beispiele 2 - 21: Haarfärbelösungen mit einem basischen pH-Wert**

| | |
|---|---|
| 10,00 g | Ethanol |
| 10,00 g | Natriumlaurylethersulfat, 28%ige Lösung in Wasser |
| 10,00 g | Ammoniak 25%ige wässrige Lösung |
| 0,30 g | Ascorbinsäure |
| 0,70 g | 3-(2,4-Diaminophenoxy)-1-propanol-sulfat |
| X g | Entwicklersubstanz gemäß Tabelle 1 |
| ad 100,00 g | Wasser, entmineralisiert |

Vor der Anwendung werden 10 Gramm der vorstehenden Haarfärbelösung mit 10 Gramm einer 6%igen wässrigen Wasserstoffperoxidlösung gemischt. Das so enthaltene Oxidationshaarfärbemittel, das eine pH-Wert zwischen 9 und 11 aufweist, wird anschliessend in der erforderlichen Menge auf gebleichte Haare aufgetragen. Nach einer Einwirkungszeit von 30 Minuten bei 40 °C wird mit einem Farbpftegeshampoo gewaschen, gespült und getrocknet. Die erhaltenen Farbnuancen und Farbintensitäten sind in Tabelle 1 zusammengefaßt.

Tabelle 1:

| **Haarfärbeispiele 2 - 21** | | | |
|---|---|---|---|
| **Beispiel** Nr. | **Entwicklersubstanz X** | **X g** | **Farbton** |
| 2 | 2-Propyl-p-phenylendiamin | 0,38 | blau |
| 3 | 2-(Hydroxymethyl)-p-phenylendiamin | 0,35 | dunkelblau |
| 4 | 2-(1-Hydroxyethyl)-p-phenylendiamin | 0,38 | violettstichig blau |
| 5 | 2-(2-Hydroxyethyl)-p-phenylendiamin | 0,38 | dunkelblau |
| 6 | 2-(1,2-Dihydroxyethyl)-p-phenylendiamin | 0,42 | blau |
| 7 | 2-(Methoxymethyl)-p-phenylendiamin | 0,42 | violettstichig blau |
| 8 | 2-(2-Hydroxyethoxy)-p-phenylendiamin | 0,42 | blau schwarz |
| 9 | 2-(3-Hydroxypropyl-p-phenylendiamin | 0,42 | blau |
| 10 | 2-(2-Thienyl)-p-phenylendiamin | 0,48 | grünstichig blau |
| 11 | 4,5-Diamino-1-methyl-1H-pyrazol | 0,28 | purpurrot |
| 12 | 4,5-Diamino-1-(2-hydroxyethyl)-1H-pyrazol | 0,36 | purpurrot |
| 13 | 4,5-Diamino-1-(1-methylethyl)-1H-pyrazol | 0,35 | purpurrot |
| 14 | 4,5-Diamino-1-(phenylmethyl)-1H-pyrazol | 0,47 | purpurrot |
| 15 | 4,5-Diamino-1-((4-methylphenyl)methyl)-1H-pyrazol | 0,51 | purpurrot |
| 16 | 4,5-Diamino-1-(2-hydroxyethyl)-3-methyl-1H-pyrazol | 0,39 | violett |
| 17 | 4,5-Diamino-1-methyl-3-phenyl-1H-pyrazol | 0,47 | blau violett |
| 18 | 2-Methyl-4-aminophenol | 0,31 | purpur |
| 19 | 3-Methyl-4-aminophenol | 0,31 | purpurrot |
| 20 | 2-Hydroxymethyl-4-aminophenol | 0,35 | rosérot |
| 21 | 3-Hydroxymethyl-4-aminophenol | 0,35 | rosé |

**Beispiele 22 - 31: Haarfärbecreme, basisch**

| | |
|---|---|
| 15,00 g | Cetylstearylalkohol 50/50 |
| 5,00 g | Glycerinmonostearat |
| 10,00 g | Natriumlaurylethersulfat, 28%ige wässrige Lösung |

(fortgesetzt)

| | |
|---|---|
| 0,30 g | Natriumsulfit |
| 5,00 g | Ammoniak, 25%ige wässrige Lösung |
| X g | Farbstoff gemäß Tabelle 3 |
| ad 100,00 g | Wasser, entmineralisiert |

Unmittelbar vor der Anwendung wird die vorstehende Färbecreme mit 100 Gramm einer 6%igen wässrigen Wasserstoffperoxidlösung vermischt. Das so erhaltene Oxidationshaarfärbemittel wird anschliessend in der notwendigen Menge auf mittelblonde Naturhaare aufgetragen. Nach einer Einwirkungszeit von 30 Minuten bei 40 °C wird das Haar mit lauwarmem Wasser gespült und sodann getrocknet.

Tabelle 2:

| **Färbeergebnisse der Beispiele 22 - 31** | |
|---|---|
| **Beispiel Nr.** | **Färbung** |
| 22 | braun schwarz |
| 23 | blau schwarz |
| 24 | rotbraun |
| 25 | blau schwarz |
| 26 | granatrot |
| 27 | rotbraun |
| 28 | violettstichig schwarz |
| 29 | braun |
| 30 | violettstichig blau |
| 31 | dunkelbraun |

**<u>Tabelle 3:</u> Haarfärbebeispiele 22 – 31** (alle Mengenangaben in Gramm/100 g Färbemittel)

| Farbstoff / Beispiel Nr. | 22 | 23 | 24 | 25 | 26 | 27 | 28 | 29 | 30 | 31 |
|---|---|---|---|---|---|---|---|---|---|---|
| 3-(2,4-Diaminophenoxy)-1-propanol-sulfat | 1,6 | | 0,02 | | | 0,1 | 0,6 | 0,6 | 0,3 | 0,05 |
| 3-(2,4-Diaminophenoxy)-1-propanol-dihydrochlorid | | 1,4 | | 0,8 | 0,6 | | | | | |
| 2-Methoxymethyl-p-phenylendiamin-dihydrochlorid | | 0,1 | | 1,0 | | | | | | |
| 2-Methyl-p-phenylendiamin-sulfat | 0,1 | 1,0 | | | | | | | | 1,0 |
| 2-(2-Hydroxyethyl)-p-phenylendiamin-sulfat | | 0,1 | | | | | 1,0 | | | |
| 2-Aminomethyl-p-phenylendiamin-dihydrochlorid | | 0,05 | | | | 0,3 | | 1,5 | | |
| 4-Amino-3-methyl-phenol | | 0,3 | | | | 0,3 | | | | |
| 4,5-Diamino-1-((4-methylphenyl)methyl)-1H-pyrazol-sulfat | 1,5 | | | | | | 0,05 | | 0,2 | |
| 4,5-Diamino-1-(2-hydroxyethyl)-1H-pyrazol-sulfat | 0,05 | | 0,1 | | 0,6 | | 0,3 | | | |
| 4,5-Diamino-1-(1-methylethyl)-1H-pyrazol-sulfat | 0,03 | | | | | 0,02 | | | | |
| 5-((2-Hydroxyethyl)amino)-2-methoxy-anilin-sulfat | 0,01 | | 0,02 | | | | | | | |
| 1-(2-Hydroxyethylamino)-3,4-methylendioxybenzol | 0,2 | | | | | | | | | |
| Resorcin | | 0,3 | | 0,2 | | | | 0,6 | | 0,3 |
| 2-Methylresorcin | 0,05 | 0,05 | | | | 0,1 | | 0,1 | | 0,2 |
| 5-Amino-2-methylphenol | 0,05 | 0,05 | | 0,2 | | | | | | |
| 3-Amino-phenol | 0,3 | 0,06 | | 0,1 | | | 0,3 | 0,1 | | 0,1 |

**Tabelle 3:** (Fortsetzung)

| **Farbstoff / Beispiel Nr.** | **22** | **23** | **24** | **25** | **26** | **27** | **28** | **29** | **30** | **31** |
|---|---|---|---|---|---|---|---|---|---|---|
| 2-Amino-6-chlor-4-nitro-phenol | 0,1 | | 0,05 | | | | | 0,2 | | |
| 2-Chlor-6-(ethylamino)-4-nitro-phenol | 0,1 | | 0,05 | | | | | 0,1 | | |
| p-Phenylendiamin | | 0,5 | | | | 0,05 | | | | |
| 2-(1-Hydroxyethyl)-p-phenylendiamin | | 0,02 | | 0,3 | | | | | | |
| 2-(2,4-Diaminophenoxy)-ethanol-sulfat | | 0,05 | 0,05 | | 0,05 | | | | | |
| N,N-Bis(2-hydroxyethyl)-p-phenylendiamin-sulfat | 0,1 | | | 0,2 | | 0,05 | | | 1,0 | |
| 4-Aminophenol | | 0,02 | | | | | | | | |
| 2,4-Diamino-1-fluor-5-methylbenzol-sulfat | | | | | 0,05 | | | | | |
| 3-Amino-6-methoxy-2-(methylamino)pyridin * 2HCl | | 0,1 | | | | | | | | |
| N-(3-(Dimethylamino)phenyl)-harnstoff | | | 0,05 | | 0,1 | | | 0,01 | | |
| 5-Amino-6-chlor-o-kresol | | 0,1 | 0,05 | | 0,05 | 0,2 | 0,1 | | | |
| 1-Naphthol | 0,08 | | | 0,1 | | 0,05 | | | 0,6 | |
| 1-Acetoxy-2-methyl-naphthalin | 0,02 | | | | | | | | | |
| 4-Chlorresorcin | | 0,1 | | | | | | 0,02 | | 0,2 |
| Sesamol | | 0,05 | | | | | | 0,02 | | 0,1 |
| 6-Amino-m-kresol | 0,05 | | | 0,03 | | 0,05 | | | | 0,02 |
| 2,6-Diamino-3-pyridin-3-yl-azo-pyridin | 0,01 | | | | | | | 0,02 | | |

**Beispiel 32: Vergleichsversuche**

Zum Nachweis der besseren Säurestabilität/Schweißstabilität der mit den erfindungsgemässen Haarfärbemitteln erhaltenen Färbungen wurden Haare, die mit einem erfindungsgemässen Mittel (Beispiel A-D) beziehungsweise einem nicht-erfindungsgemässen Mittel (Beispiel A'-D') gefärbt wurden, mit einer mit Milchsäure auf pH 3,2 eingestellten Lösung von 10% Natriumchlorid, 1% Dikaliumhydrogenphosphat und 0,25% Histidin 48 Stunden lang bei 37 °C behandelt. Die Haare wurden anschliessend mit Wasser ausgespült und getrocknet.
Der erhaltene Farbton wurde anschliessend jeweils mit einem Farbmessgerät der Firma Minolta, Typ Chromameter II, im Lab-System gemessen und mit den entsprechenden Farbmesswerten der gefärbten Haare vor der Säurebehandlung verglichen. Hierbei steht der L-Wert für die Helligkeit (das heißt je geringer der L-Wert ist, umso grösser ist die Farbintensität), während der a-Wert ein Maß für den Rotanteil ist (das heißt je grösser der a-Wert ist, umso grösser ist der Rotanteil). Der b-Wert ist ein Maß für den Blauanteil der Farbe, wobei der Blauanteil umso grösser ist, je negativer der b-Wert ist.
Als Mass für den Stabilität der Färbung wurde die Gesamtfarbänderung ΔE der Haarsträhnchen bei der Behandlung mit Säure nach folgender Formel ermittelt:

$$\Delta E = \sqrt{(L_0 - L_s)^2 + (a_0 - a_s)^2 + (b_0 - b_s)^2}$$

$L_0$: Helligkeit vor der Behandlung
$L_s$: Helligkeit nach der Behandlung
$a_0$: Rot-Grün-Wert vor der Behandlung
$a_s$: Rot-Grün-Wert nach der Behandlung
$b_0$: Gelb-Blau-Wert vor der Behandlung
$b_s$: Gelb-Blau-Wert nach der Behandlung

**Haarfärbemittel**

| | |
|---|---|
| 10,00 g | Ethanol |
| 10,00 g | Natriumlaurylethersulfat, 28%ige Lösung in Wasser |
| 10,00 g | Ammoniak 25%ige wässrige Lösung |
| 0,30 g | Ascorbinsäure |
| 2,5 mmol | Kupplersubstanz gemäß Tabelle 4 |
| 2,5 mmol | Entwicklersubstanz gemäß Tabelle 4 |
| ad 100,00 g | Wasser, entmineralisiert |

Tabelle 4:

| Beispiel | Kupplersubstanz | Entwicklersubstanz |
|---|---|---|
| **A** (erfindungsgemäss) | 3-(2,4-Diaminophenoxy)-1-propanol-dihydrochlorid | 2-(2-Hydroxyethyl)-p-phenylendiaminsulfat |
| A' | 2-(2,4-Diaminophenoxy)-ethanol-dihydrochlorid | 2-(2-Hydroxyethyl)-p-phenylendiaminsulfat |
| B (erfindungsgemäss) | 3-(2,4-Diaminophenoxy)-1-propanol-dihydrochlorid | 4,5-Diamino-1-(2-hydroxyethyl)-1H-pyrazol-sulfat |
| **B`** | 2-(2,4-Diaminophenoxy)-ethanol-dihydrochlorid | 4,5-Diamino-1-(2-hydroxyethyl)-1H-pyrazol-sulfat |
| C (erfindungsgemäss) | 3-(2,4-Diaminophenoxy)-1-propanol-sulfat | 2-(2-Hydroxyethyl)-p-phenylendiaminsulfat |
| C' | 2-(2,4-Diaminophenoxy)-ethanol-sulfat | 2-(2-Hydroxyethyl)-p-phenylendiaminsulfat |

Tabelle 4: (fortgesetzt)

| Beispiel | Kupplersubstanz | Entwicklersubstanz |
|---|---|---|
| D (erfindungsgemäss) | 3-(2,4-Diaminophenoxy)-1-propanol-sulfat | 4,5-Diamino-1-(2-hydroxyethyl)-1H-pyrazol-sulfat |
| D' | 2-(2,4-Diaminophenoxy)-ethanol-sulfat | 4,5-Diamino-1-(2-hydroxyethyl)-1H-pyrazol-sulfat |

**Tabelle 5: Farbmesswerte**

| Beispiel | $L_0$ | $a_0$ | $b_0$ | $L_s$ | $a_s$ | $b_s$ | $\Delta E$ |
|---|---|---|---|---|---|---|---|
| A | 17,16 | +3,73 | -8,88 | 25,02 | +6,52 | -5,52 | 9,0 |
| A' | 16,01 | +2,30 | -5,20 | 24,40 | +7,45 | -5,12 | 9,8 |
| B | 19,67 | +24,27 | +4,52 | 28,00 | +25,69 | +5,51 | 8,5 |
| B' | 19,47 | +23,84 | +4,13 | 33,25 | +29,40 | +5,21 | 14,9 |
| C | 15,66 | +1,66 | -3,48 | 21,80 | +6,12 | -2,58 | 7,6 |
| C' | 17,03 | +2,96 | -6,86 | 27,01 | +6,21 | -3,55 | 11,0 |
| D | 19,72 | +24,21 | +4,44 | 25,57 | +27,65 | +4,84 | 6,8 |
| D' | 20,45 | +29,76 | +5,31 | 30,85 | +30,02 | +3.75 | 10,5 |

Alle Prozentangaben stellen soweit nicht anders angegeben Gewichtsprozente dar.

## Patentansprüche

**1.** Mittel zur Färbung von Keratinfasern auf der Basis einer Entwicklersubstanz-Kupplersubstanz-Kombination, welches **dadurch gekennzeichnet ist, dass** es als Kupplersubstanz 3-(2,4-Diaminophenoxy)-1-propanol und als Entwicklersubstanz

- ein am Benzolring monosubstituiertes p-Phenylendiaminderivat der allgemeinen Formel (I),

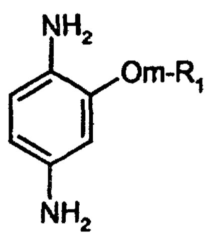

(I)

,

worin m gleich 0 oder 1 ist und $\mathbf{R_1}$ einen geradkettigen oder verzweigten $C_3$-$C_6$-Alkylrest, einen geradkettigen oder verzweigten Monohydroxy-($C_1$-$C_6$)-alkylrest oder Polyhydroxy-($C_2$-$C_6$)alkylrest, einen geradkettigen oder verzweigten Mono-($C_2$-$C_6$)-alkoxy-($C_2$-$C_6$)-alkylrest oder Poly-($C_2$-$C_6$)-alkoxy-($C_2$-$C_6$)-alkylrest oder eine carbozyklische oder heterozyklische, substituierte oder unsubstituierte, aromatische Verbindung darstellt; und/oder

- ein 4,5-Diamino-1H-Pyrazolderivat gemäß der allgemeinen Formel (II)

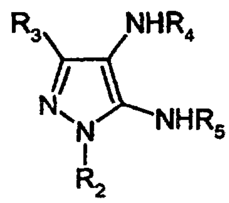

(II)

,

worin $R_2$, $R_4$ und $R_5$ unabhängig voneinander ein Wasserstoffatom oder einen geradkettigen oder verzweigten $C_1$-$C_6$-Alkylrest, einen geradkettigen oder verzweigten Monohydroxy-($C_1$-$C_6$)-alkylrest oder Polyhydroxy-($C_2$-$C_6$)-alkylrest oder einen unsubstituierten oder am Aromaten substituierten Benzylrest darstellen, und $R_3$ gleich einem geradkettigen oder verzweigten $C_1$-$C_6$-Alkylrest, einem geradkettigen oder verzweigten Monohydroxy-($C_1$-$C_6$)-alkylrest oder Polyhydroxy-($C_2$-$C_6$)-alkylrest, einem geradkettigen oder verzweigten Mono-($C_2$-$C_6$)-alkoxy-($C_2$-$C_6$)-alkylrest oder Poly-($C_2$-$C_6$)-alkoxy-($C_2$-$C_6$)-alkylrest oder einer carbozyklischen oder heterozyklischen, substituierten oder unsubstituierten, aromatischen Verbindung ist, oder $R_2$ gleich einer Methylgruppe, einer isopropylgruppe, einer 2-Hydroxyethylgruppe, einer Benzylgruppe oder einer 4-Methyl-benzylgruppe ist, wenn gilt **$R_3$=$R_4$=$R_5$=** Wasserstoff;
und/oder

- ein p-Aminophenolderivat der allgemeinen Formel (III)

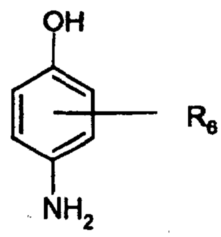

(III)

,

worin $R_6$ gleich einem geradkettigen oder verzweigten $C_1$-$C_6$-Alkylrest, einem geradkettigen oder verzweigten Monohydroxy-($C_1$-$C_6$)-alkylrest oder Polyhydroxy-($C_2$-$C_6$)-alkylrest, einem geradkettigen oder verzweigten Mono-($C_2$-$C_6$)-alkoxy-($C_2$-$C_6$)-alkylrest oder Poly-($C_2$-$C_6$)-alkoxy-($C_2$-$C_6$)-alkylrest, einem geradkettigen oder verzweigten Amino-($C_2$-$C_6$)-Alkylrest oder einer carbozyklischen oder heterozyklischen, substituierten oder unsubstituierten, aromatischen Verbindung ist, enthält.

**2.** Mittel nach Anspruch 1, **dadurch gekennzeichnet, dass** das p-Phenylendiaminderivat der Formel (I) ausgewählt ist aus 2-Propyl-p-phenylendiamin, 2-(Hydroxymethyl)-p-phenylendiamin, 2-(1-Hydroxyethyl)-p-phenylendiamin, 2-(2-Hydroxyethyl)-p-phenylendiamin, 2-(3-Hydroxypropyl)-p-phenylendiamin, 2-(1,2-Dihydroxyethylp-phenylendiamin, 2-(Methoxymethyl)-p-phenylendiamin, 2-(2-Methoxyethyl)-p-phenylendiamin, 2-(2-Hydroxyethoxy)-p-phenylendiamin, 2,5-Diaminobiphenyl, 2-(2-Thienyl)-p-phenylendiamin, 2-(3-Thienyl)-p-phenylendiamin und 3-(2,5-Diaminophenyl)pyridin.

**3.** Mittel nach Anspruch 1, **dadurch gekennzeichnet, dass** das Pyrazolderivat der Formel (II) ausgewählt ist aus 4,5-Diamino-1-methyl-1H-pyrazol, 4,5-Diamino-1-(2-hydroxy-ethyl)-1H-pyrazol, 4,5-Diamino-1-(1-methylethyl)-1H-pyrazol, 4,5-Diamino-1-(phenylmethyl)-1H-pyrazol, 4,5-Diamino-1-((4-methylphenyl)methyl)-1H-pyrazol, 4,5-Diamino-1-(2-hydroxyethyl)-3-methyl-1H-pyrazol und 4,5-Diamino-1-methyl-3-phenyl-1H-pyrazol.

**4.** Mittel nach Anspruch 1, **dadurch gekennzeichnet, dass** das p-Aminophenolderivat der Formel (III) ausgewählt ist aus3-Methyl-pamino-phenol, 2-Aminomethyl-p-amino-phenol, 2-Hydroxymethyl-p-amino-phenol, 3-Hydroxymethyl-p-amino-phenol, 2-Phenyl-p-aminophenol, 2-(2-Thienyl)-p-aminophenol, 2-(3-Thienyl)-p-aminophenol, 3-(2-Thienyl)-p-aminophenol und 3-(3-Thienyl)-p-aminophenol.

**5.** Mittel nach einem der Ansprüche 1 bis 4, **dadurch gekennzeichnet, dass** es zusätzlich weitere Entwicktersubstanzen und/oder Kupplersubstanzen und/oder direktziehende Farbstoffe enthält.

**6.** Mittel nach einem der Ansprüche 1 bis 5, **dadurch gekennzeichnet, dass** es das 3-(2,4-Diamino-phenoxy)-1-propanol sowie die Verbindungen der Formel (I) bis (III) jeweils in einer Menge von 0,01 bis 5 Gewichtsprozent enthält

**7.** Mittel nach einem der Ansprüche 1 bis 6, **dadurch gekennzeichnet, dass** es einen pH-Wert von 6,8 bis 11,5 aufweist.

**8.** Mittel nach einem der Ansprüche 1 bis 7, **dadurch gekennzeichnet, dass** es ein Haarfärbemittel ist.

**9.** Verfahren zum oxidativen Färben von Haaren, **dadurch gekennzeichnet, dass** man vor der Anwendung ein Haarfärbemittel nach einem der Ansprüche 1 bis 8 mit einem Oxidationsmittel vermischt und auf das Haar aufträgt, bei einer Temperatur von 15 bis 50°C 10 bis 45 Minuten lang einwirken lässt, das Haar anschliessend mit Wasser ausspült, gegebenenfalls shampooniert und sodann trocknet.

## Claims

**1.** Agent for colouring keratinic fibres based on a developer substance-coupler substance combination which is **characterized in that** it comprises, as coupler substance, 3-(2,4-diaminophenoxy)-1-propanol and, as developer substance,

- a p-phenylenediamine derivative monosubstituted on the benzene ring and of the general formula (I),

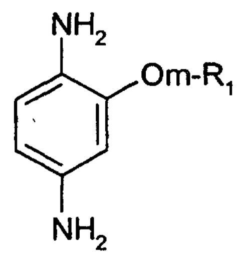

(I)

in which m is 0 or 1 and $R_1$ is a straight-chain or branched $C_3$-$C_6$-alkyl radical, a straight-chain or branched monohydroxy($C_1$-$C_6$)alkyl radical or polyhydroxy($C_2$-$C_6$)alkyl radical, a straight-chain or branched mono($C_2$-$C_6$) alkoxy ($C_2$-$C_6$) alkyl radical or poly($C_2$-$C_6$)alkoxy($C_2$-$C_6$)alkyl radical or a carbocyclic or heterocyclic, substituted or unsubstituted, aromatic compound;
and/or

- a 4,5-diamino-1H-pyrazole derivative according to the general formula (II)

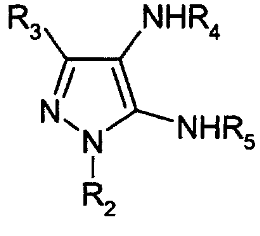

(II)

in which $R_2$, $R_4$ and $R_5$, independently of one another, are a hydrogen atom or a straight-chain or branched $C_1$-$C_6$-alkyl radical, a straight-chain or branched monohydroxy($C_1$-$C_6$)alkyl radical or polyhydroxy($C_2$-$C_6$)-alkyl radical or a benzyl radical which is unsubstituted or substituted on the aromatic, and $R_3$ is a straight-chain or branched $C_1$-$C_6$-alkyl radical, a straight-chain or branched monohydroxy ($C_1$-$C_6$)alkyl radical or polyhydroxy ($C_2$-$C_6$)alkyl radical, a straight-chain or branched mono ($C_2$-$C_6$) alkoxy ($C_2$-$C_6$) alkyl radical or poly($C_2$-$C_6$) alkoxy($C_2$-$C_6$)alkyl radical or a carbocyclic or heterocyclic, substituted or unsubstituted, aromatic compound or $R_2$ is a methyl group, an isopropyl group, a 2-hydroxyethyl group, a benzyl group or a 4-methylbenzyl group, when $R_3$=$R_4$=$R_5$= hydrogen;
and/or

- a p-aminophenol derivative of the general formula (III)

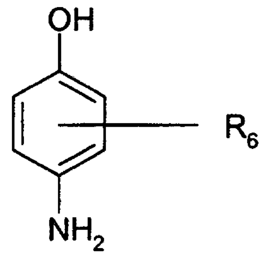

(III)

in which $R_6$ is a straight-chain or branched $C_1$-$C_6$-alkyl radical, a straight-chain or branched monohydroxy ($C_1$-$C_6$) alkyl radical or polyhydroxy($C_2$-$C_6$)alkyl radical, a straight-chain or branched mono ($C_2$-$C_6$) alkoxy ($C_2$-$C_6$) alkyl radical or poly ($C_2$-$C_6$) alkoxy ($C_2$-$C_6$) alkyl radical, a straight-chain or branched amino($C_2$-$C_6$) alkyl radical or a carbocyclic or heterocyclic, substituted or unsubstituted, aromatic compound.

2. Agent according to Claim 1, **characterized in that** the p-phenylenediamine derivative of the formula (I) is chosen from 2-propyl-p-phenylenediamine, 2-(hydroxymethyl)-p-phenylenediamine, 2-(1-hydroxyethyl)-p-phenylenediamine, 2-(2-hydroxyethyl)-p-phenylenediamine, 2-(3-hydroxypropyl)-p-phenylenediamine, 2-(1,2-di-hydroxyethyl)-p-phenylenediamine, 2-(methoxymethyl)-p-phenylenediamine, 2-(2-methoxyethyl)-p-phenylenediamine, 2-(2-hydroxyethoxy)-p-phenylenediamine, 2,5-diaminobiphenyl, 2-(2-thienyl)-p-phenylenediamine, 2-(3-thienyl)-p-phenylenediamine and 3-(2,5-diaminophenyl)pyridine.

3. Agent according to Claim 1, **characterized in that** the pyrazole derivative of the formula (II) is chosen from 4,5-diamino-1-methyl-1H-pyrazole, 4,5-diamino-1-(2-hydroxyethyl)-1H-pyrazole, 4,5-diamino-1-(1-methylethyl)-1H-pyrazole, 4,5-diamino-1-(phenylmethyl)-1H-pyrazole, 4,5-diamino-1-((4-methylphenyl)methyl)-1H-pyrazole, 4,5-diamino-1-(2-hydroxyethyl)-3-methyl-1H-pyrazole and 4,5-diamino-1-methyl-3-phenyl-1H-pyrazole.

4. Agent according to Claim 1, **characterized in that** the p-aminophenol derivative of the formula (III) is chosen from 3-methyl-p-aminophenol, 2-aminomethyl-p-aminophenol, 2-hydroxymethyl-p-aminophenol, 3-hydroxymethyl-p-aminophenol, 2-phenyl-p-aminophenol, 2-(2-thienyl)-p-aminophenol, 2-(3-thienyl)-p-aminophenol, 3-(2-thienyl)-p-aminophenol and 3-(3-thienyl)-p-aminophenol.

5. Agent according to one of Claims 1 to 4, **characterized in that** it additionally comprises further developer substances and/or coupler substances and/or direct dyes.

6. Agent according to one of Claims 1. to 5, **characterized in that** it comprises the 3-(2,4-diaminophenoxy)-1-propanol and the compounds of the formula (I) to (III) in each case in an amount of from 0.01 to 5 per cent by weight.

7. Agent according to one of Claims 1 to 6, **characterized in that** it has a pH of from 6.8 to 11.5.

8. Agent according to one of Claims 1 to 7, **characterized in that** it is a hair colorant.

9. Method for the oxidative colouring of hair, **characterized in that**, prior to application, a hair colorant according to one of Claims 1 to 8 is mixed with an oxidizing agent and applied to the hair, left to act at a temperature of from 15 to 50°C for 10 to 45 minutes, the hair is then rinsed with water, optionally shampooed and then dried.

**Revendications**

1. Composition pour la teinture de fibres de kératine, à base d'une association de développeur-coupleur, qui est **caractérisée en ce qu'**elle contient en tant que coupleur du 3-(2,4-diaminophénoxy)-1-propanol et en tant que développeur

- un dérivé de p-phénylènediamine monosubstitué sur le cycle benzénique, de formule générale (I)

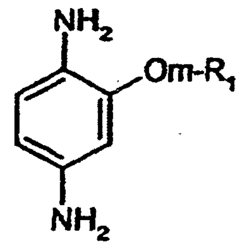

(I)

dans laquelle m est égal à 0 ou 1 et **$R_1$** représente un radical alkyle en $C_3$-$C_6$ à chaîne droite ou ramifié, un radical monohydroxyalkyle($C_1$-$C_6$) ou polyhydroxyalkyle($C_2$-$C_6$) à chaîne droite ou ramifié, un radical monoalcoxy ($C_2$-$C_6$) -alkyle ($C_2$-$C_6$) ou polyalcoxy($C_2$-$C_6$)-alkyle($C_2$-$C_6$) à chaîne droite ou ramifié, ou un composé aromatique carbocyclique ou hétérocyclique, substitué ou non substitué ;
et/ou

- un dérivé de 4,5-diamino-1H-pyrazole de formule générale (II)

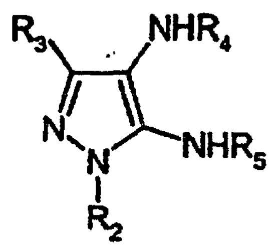

(II)

dans laquelle **$R_2$**, **$R_4$** et **$R_5$** représentent, indépendamment les uns des autres, un atome d'hydrogène ou un radical alkyle en $C_1$-$C_6$ à chaîne droite ou ramifié, un radical monohydroxyalkyle-($C_1$-$C_6$) ou polyhydroxyalkyle ($C_2$-$C_6$) à chaîne droite ou ramifié, ou un radical benzyle non substitué ou substitué sur le noyau aromatique, et **$R_3$** représente un radical alkyle en $C_1$-$C_6$ à chaîne droite ou ramifié, un radical monohydroxyalkyle($C_1$-$C_6$) ou polyhydroxyalkyle($C_2$-$C_6$) à chaîne droite ou ramifié, un radical monoalcoxy($C_2$-$C_6$)-alkyle ($C_2$-$C_6$) ou polyalcoxy ($C_2$-$C_6$) -alkyle ($C_2$-$C_6$) à chaîne droite ou ramifié, ou un composé aromatique carbocyclique ou hétérocyclique, substitué ou non substitué, ou **$R_2$** représente un groupe méthyle, un groupe isopropyle, un groupe 2-hydroxyéthyle, un groupe benzyle ou un groupe 4-méthyl-benzyle, lorsque **$R_3$** = **$R_4$ = $R_5$ =** un atome d'hydrogène ;
et/ou

- un dérivé de p-aminophénol de formule générale (III)

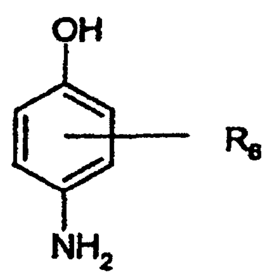

(III)

dans laquelle **$R_6$** représente un radical alkyle en $C_1$-$C_6$ à chaîne droite ou ramifié, un radical monohydroxyalkyle ($C_1$-$C_6$) ou polyhydroxyalkyle-($C_2$-$C_6$) à chaîne droite ou ramifié, un radical monoalcoxy($C_2$-$C_6$)-alkyle($C_2$-$C_6$) ou polyalcoxy-($C_2$-$C_6$) -alkyle ($C_2$-$C_6$) à chaîne droite ou ramifié, un radical aminoalkyle($C_2$-$C_6$) à chaîne droite ou ramifié, ou un composé aromatique carbocyclique ou hétérocyclique, substitué ou non substitué.

2. Composition selon la revendication 1, **caractérisée en ce que** le dérivé de p-phénylènediamine de formule (I) est choisi parmi la 2-propyl-p-phénylènediamine, la 2-(hydroxyméthyl)-p-phénylènediamine, la 2-(1-hydroxyéthyl)-p-phénylènediamine, la 2-(2-hydroxyéthyl)-p-phénylènediamine, la 2-(3-hydroxypropyl)-p-phénylènediamine, la 2-(1,2-dihydroxyéthyl)-p-phénylènediamine, la 2-(méthoxyméthyl)-p-phénylènediamine, la 2-(2-méthoxyéthyl)-p-phénylènediamine, la 2-(2-hydroxyéthoxy)-p-phénylènediamine, le 2,5-diaminobiphényle, la 2-(2-thiényl)-p-phénylènediamine, la 2-(3-thiényl)-p-phénylènediamine et la 3-(2,5-diaminophényl)pyridine.

**3.** Composition selon la revendication 1, **caractérisée en ce que** le dérivé de pyrazole de formule (II) est choisi parmi le 4,5-diamino-1-méthyl-1H-pyrazole, le 4,5-diamino-1-(2-hydroxy-éthyl)-1H-pyrazole, le 4,5-diamino-1-(1-méthyléthyl)-1H-pyrazole, le 4,5-diamino-1-(phénylméthyl)-1H-pyrazole, le 4,5-diamino-1-((4-méthylphényl)-méthyl)-1H-pyrazole, le 4,5-diamino-1-(2-hydroxyéthyl)-3-méthyl-1H-pyrazole et le 4,5-diamino-1-méthyl-3-phényl-1H-pyrazole.

**4.** Composition selon la revendication 1, **caractérisée en ce que** le dérivé de p-aminophénol de formule (III) est choisi parmi le 3-méthyl-p-aminophénol, le 2-aminométhyl-p-aminophénol, le 2-hydroxyméthyl-p-aminophénol, le 3-hydroxyméthyl-p-aminophénol, le 2-phényl-p-aminophénol, le 2-(2-thiényl)-p-aminophénol, le 2-(3-thiényl)-p-aminophénol, le 3-(2-thiényl)-p-aminophénol et le 3-(3-thiényl)-p-aminophénol.

**5.** Composition selon l'une quelconque des revendications 1 à 4, **caractérisée en ce qu'**elle contient en outre d'autres développeurs et/ou coupleurs et/ou des colorants directs.

**6.** Composition selon l'une quelconque des revendications 1 à 5, **caractérisée en ce qu'**elle contient du 3-(2,4-diamino-phénoxy)-1-propanol ainsi que les composés de formules (I) à (III) chacun en une quantité allant de 0,01 à 5 % en poids.

**7.** Composition selon l'une quelconque des revendications 1 à 6, **caractérisée en ce qu'**elle présente un pH de 6,8 à 11,5.

**8.** Composition selon l'une quelconque des revendications 1 à 7, **caractérisée en ce qu'**elle est une composition de teinture pour cheveux.

**9.** Procédé pour la teinture des cheveux par oxydation, **caractérisé en ce que**, avant l'emploi, on mélange avec un oxydant une composition de teinture pour cheveux selon l'une quelconque des revendications 1 à 8 et on l'applique sur les cheveux, on la laisse agir pendant 10 à 45 minutes à une température de 15 à 50°C, puis on rince les cheveux à l'eau, éventuellement on les shampooine et ensuite on les sèche.